# EUROPEAN PATENT SPECIFICATION

(11) **EP 1 684 728 B1**
(45) Date of publication and mention of the grant of the patent: **16.09.2009**
(21) Application number: 04761983.8
(22) Date of filing: 29.10.2004
(51) Int. Cl.: A61K 9/20, A61K 31/192

(54) **NON-EFFERVESCENT FORM OF SODIUM NAPROXEN COMPRISING I.A. SODIUM HYDROGEN CARBONATE**
NICHTSPRUDELNDE FORM VON NATRIUM-NAPROXEN MIT NATRIUMHYDROGENCARBONAT U.A.
FORME NON EFFERVESCENTE DE NAPROXENE DE SODIUM COMPRENANT DU BICARBONATE DE SOUDE

(30) Priority: 30.10.2003 CH 184803
(43) Date of publication of application: 02.08.2006
(73) Proprietor: Bayer Consumer Care AG, 4052 Basel (CH)
(72) Inventor: GRUBER, Peter, 79249 Merzhausen (DE); SIEGMUND, Martin, 79618 Rheinfelden (DE)
(74) Representative: Bohest AG
(86) International application number: PCT/CH2004/000655
(87) International publication number: WO 2005/041938

(56) References cited:
- WO-A-97/30699
- WO-A-02/083105
- WO-A-02/083110
- US-A- 5 034 416
- US-A- 6 165 506
- US-B1- 6 284 274

## Description

The invention relates to a non-effervescent tablet formulation for oral administration of sodium naproxen and a process for the production thereof.

Naproxen, i.e. (S)-2-(6-methoxy-2-naphthyl)propionic acid is a known medicine with analgesic, antiphlogistic and antipyretic properties, that in particular is employed for the treatment of inflammatory diseases and against pain, such as rheumatic diseases, headaches, migraines, toothaches, back aches, muscle pain, post-operative pain and the like. The extended effect of naproxen with protracted headaches and ongoing muscle and limb pain is an especial advantage.

A further essential point, especially in pain treatment, is the achievement of a rapid onset of the effect. In order to reach this, the active ingredient must be rapidly released and absorbed, which in the case of solid dosage forms further requires that these rapidly disintegrate in the gastrointestinal tract. On the other hand, the solid dosage forms should be small enough that they can still be swallowed without problem.

The formulations must however contain suitable auxiliary agents in sufficient quantities, so that the formulations can be compressed in the usual tabletization machinery, do not stick to tabletization tools and result in rapidly disintegrating tablets with sufficient hardness. Moreover, the achievement of a rapid onset of the effect is made more difficult by the fact that naproxen is virtually insoluble in acidic media, in particular in gastric acid, whereby the dissolution and resorption of the active ingredient can be considerably delayed.

Therefore, there have been attempts to improve the solubility of naproxen acid.

In WO 97/18245 therefore a complex of naproxen (acidic form) and β-cyclodextrin was produced. The ratio between the active ingredient and the β-cyclodextrin is however so unfavourable that no swallowable tablets can be produced from it. In addition it is not certain whether in vivo the naproxen is sufficiently rapidly dissolved out of the β-cyclodextrin-complex and is absorbed. In any case it must be assumed that the release of the naproxen from the complex is subject to considerable fluctuations depending on the pH conditions, the ion concentrations etc. in the gastrointestinal tract.

In DE 4410470 a composition of naproxen with 0.8 - 1.5 mol arginine and 0 - 0.7 mol of basic auxiliary agent is described, each based on 1 mol naproxen. Preferred drug forms consist of granulates, which are dissolved in water before they are taken. Through the basic pH of the solution, a corresponding salt gradually forms from the naproxen. This dissolution process definitely does not take place in this way with a corresponding tablet in the stomach in the presence of gastric acid. Since the auxiliary agents are highly water soluble, they are preferably buffered from the gastric acid, before they bring the poorly soluble naproxen into solution. In addition such a formulation leads to very large, difficult to swallow tablets and is very expensive as a consequence of the addition of 0.8 - 1.5 mol equivalent arginine.

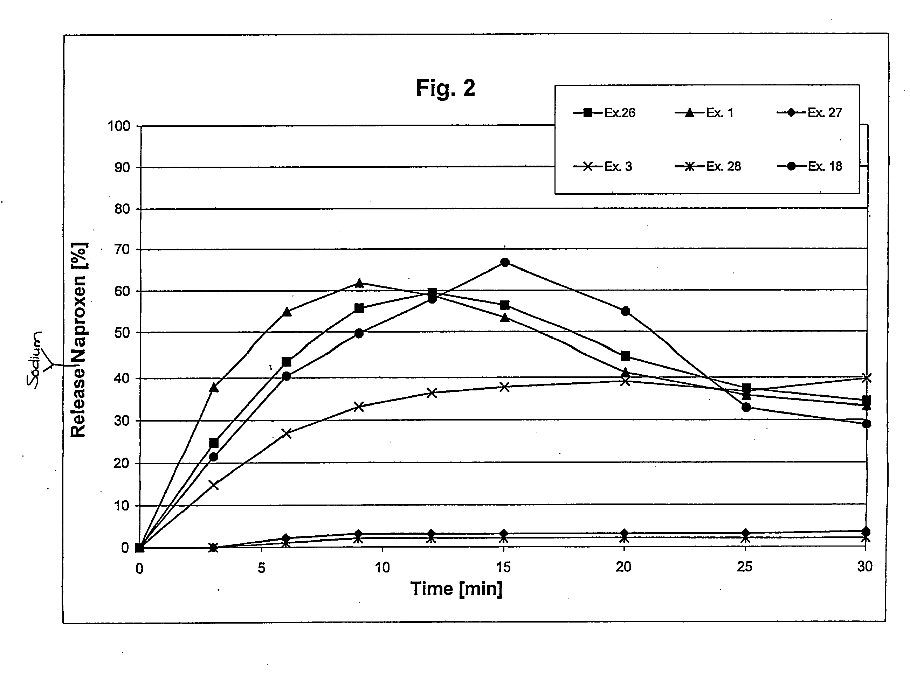

Therefore there are also film tablets already available on the market, which contain the sodium salt of naproxen in the presence of customary auxiliary agents in the tablet core, such as microcrystalline cellulose, disintegrants and magnesium stearate. The active ingredient release is however very poor at pH 1.2 (cf. Fig. 2 and Example 27).

In addition it has been tried to further improve the compressibility and the solubility of the naproxen through spray drying of naproxen or sodium naproxen (US 5470580). Also, however, tablets with sodium naproxen and spray dried mannitol (CA 2363528) have been created, in which the spraydried mannitol likewise supposedly improves the dissolution process of the active ingredient.

In US 2002/187195 soft gelatine capsules are described, which contain polyethylene glycol, sodium propionate and a co-solvent such as dimethylisosorbide. Aspirin or naproxen are named as preferred active ingredients.

WO0013672 (NANOSYSTEMS, US) discloses an effervescent solid dose nanoparticulate naproxen formulation having a high rate of dissolution comprising: (a) naproxen having an effective average particle size of less than about 600 nm; (b) a surface modifier adsorbed on the surface thereof, and (c) a pharmaceutically acceptable alkali agent, wherein the alkali agent functions to increase the dissolution rate of the nanoparticulate naproxen following administration.

EP0274734 (PHARMIDEA SRL) refers to a tablet for pharmaceutical use able to release active substance at successive times, comprising at least: a first layer containing a portion of the active substance with suitable excipients, a barrier layer of polymer material gellable and/or soluble in water and/or aqueous liquids, which is interposed between said first layer and a third layer containing the remaining portion of active substance with suitable excipients, said barrier layer and said third layer being housed in a casing consisting of polymer material impermeable and insoluble in water and/or soluble in an alkaline environment.

All of the above described formulations have, as well as the already mentioned disadvantages, the main disadvantage that the dependence of the dissolution process on the physiological conditions in the stomach and the achievement of a reproducible dissolution have been paid little attention. Thus, for example, sodium naproxen precipitates immediately in the presence of acid as a fatty hydrophobic mass, which delays the further disintegration process of the tablet core, as well as the dissolution process of the active ingredient. Instead the fatty, precipitated, hydrophobic acid form of naproxen gradually forms naproxen crystals, which however go too slowly into solution in the transition into the duodenum and the resultant pH increase in there. While naproxen rapidly goes into solution at pH 7.4 through salt formation, pH values of 7 are however not achieved in the duodenum. This leads to the fact that the naproxen is gradually dissolved only in the deeper intestinal regions and thereby a rapid build up of the active ingredient level is not possible.

The object of this invention is therefore to provide a technically feasibly manufacturable tablet formulation, that permits a rapid release and resorption of the active ingredient and that nevertheless allows comparatively small tablet sizes.

The object is achieved through a claim 1.

The sodium salt of naproxen can be present in the tablet formulations according to the invention in essentially water free form or in the form of a hydrate, e.g. as dihydrate; typically the water content of the sodium salt of naproxen can be approximately 0.05 to 14% by weight, based on the weight of the hydrate. The auxiliary agent component can contains one or more basic auxiliary agents, their total amount, based on the weight of the tablet core, is at least about 5% by weight.

Surprisingly it was found that the ability of sodium naproxen to be tabletized depends on its water content and, contrary to current opinion, it is possible to produce tablets with sufficient hardness and short disintegration times, that in addition to sodium naproxen only must contain a low proportion of basic auxiliary agent, if a sodium naproxen is used with a water content of 0.05 to 14% by weight, preferably 6 to 12.5% by weight and the water content is precisely controlled. Due to the poor compression properties and the waxy nature, a person skilled in the art would normally never try to produce a tablet that is largely free of auxiliary agent, but would add comparatively high quantities of compressible fillers and disintegrants, in order to obtain, nevertheless, useful compression and disintegration properties. It was therefore completely unexpected, that by means of suitable water content, to permit to produce tablets which almost exclusively consist of sodium naproxen and contain only a very low proportion of basic auxiliary agent.

In graphical form
- Fig. 1: shows the hardness and the disintegration time of a tablet according to the invention in relation to the compression force used in the tabletization process,
- Fig. 2: shows the dissolution profile of tablets according to the invention and comparative formulations in 0.1 M hydrochloric acid (pH 1.2) according to the Paddle-Method at 50 rpm.

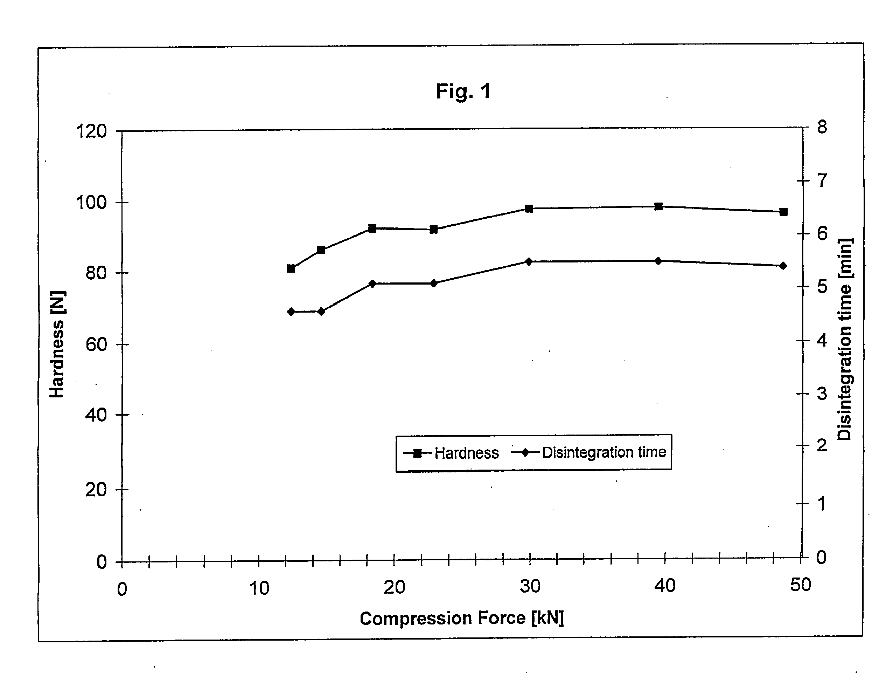

In principle sodium naproxen can be practically water free, or can exist as the monohydrate or dihydrate, or as a mixture of these forms. The water free form and the monohydrate are hygroscopic and take up water, resulting in the formation of the dihydrate. For example, water free sodium naproxen spontaneously takes up to about 12.5% by weight of water already at a relative humidity level of 43% RH. Therefore, if the anhydrate or monohydrate were used, a hygroscopic tablet would therefore result, which would need a very thick packing material. Surprisingly, it was furthermore found that the hardness and disintegration time of the tablets according to the invention, also in the absence of a classic disintegrant, are largely independent of the compression pressure used in the tabletization. Figure 1 illustrates in graphical form the hardness measured by means of a Schleuniger Hardness Tester and the disintegration time measured in water at 37°C in relation to the compression force used for a tablet according to the invention, consisting of 251.4 mg (corresponding to 220 mg water free sodium naproxen) sodium naproxen diydrate (water content of 12.5 to 14% by weight), 50 mg polyvinylpyrrolidone K25 and 50 mg sodium hydrogen carbonate. As is apparent, an increase of the compression force used from 20 to 50 kN only leads to an insignificant increase of the hardness and the disintegration time. Owing to this unexpected finding, it can be practically ruled out that tablets which are too hard and with impaired disintegration and release properties could result from the use of too much pressure, which additionally facilitates the production of the tablets according to the invention.

Furthermore, it was unexpectedly found that the tablets according to the invention can be produced without the addition of an inner lubricant such as magnesium stearate, calcium stearate, stearic acid, fat triglycerides and the like. As is known, lubricants must usually be added to the tablet mixtures, so that there is no sticking to the tabletization tools and so that the friction is not too great when the tablet is ejected. Without the use of a lubricant, considerable disturbance to the tabletization process normally results, which has the consequence that the tablet press must be turned off and the tablets are unusable, as they are injured by the ejection from the machinery. It was therefore completely surprising that lubricants could be dispensed with in the production of tablets according to the invention and that by using customary tablet presses, millions of tablets could be pressed without any addition of an inner lubricant. Moreover, the customary lubricants are hydrophobic and decrease the compressibility and the disintegration properties. Therefore, the tablet formulations according to the invention preferably do not contain significant quantities (i.e. less than 0.1% by weight) of lubricants in the tablet core, and they are advantageously completely free of inner lubricants.

If however the sodium naproxen possesses only a very low water content (of e.g. less than about 1% by weight), it is advisable to add in about 0.1 to 5% by weight of lubricant and/or glidant, based on the weight of the tablet core. Typically in such cases the proportion of lubricant (such as magnesium stearate, calcium stearate, stearic acid of fattriglyceride) can be 0.5 to 1.0 % by weight and the proportion of glidant (e.g. talcum) about 2 to 3% by weight.

Further with the elimination of inner lubricants, it has turned out that it is also not required to add a disintegrant to the tablet mix. The proportion of auxiliary agents can thereby be further reduced or fillers can even be completely eliminated. The water solubility of the sodium naproxen is actually so great, that the disintegration of the tablet can hardly be further improved through the addition of customary disintegrants or combinations of fillers such as microcrystalline cellulose with disintegrants. Therefore, the tablet formulations according to the invention preferably do not contain significant quantities (i.e. less than 0.1% by weight) of disintegrants or fillers with disintegrant properties, such as crosslinked polyvinylpyrrolidones, magnesium aluminium silicates, microcrystalline cellulose, starches, sodium carboxymethylcellulose starches etc., and advantageously they are completely free of such materials.

If the sodium naproxen has only a very low water content, it is advisable to also use in addition to lubricants and/or glidants auxiliary agents which improve the compressibility to tablets, such as microcrystalline cellulose.

The disintegration times of the tablets according to the invention are generally significantly below 10 minutes, typically in the range from about 2 to 7 minutes. Owing to the high water solubility of the sodium naproxen and the elimination of an inner lubricant, the tablets according to the invention enable a particularly rapid release and resorption of the active ingredient, which leads to a rapid increase of the blood level and concentration at the site of effect. Furthermore, it was found that tablets according to the invention which contain at least about 5% by weight of basic component can lead to significantly supersaturated solutions in acidic medium, which additionally aids a rapid resorption. In comparison to known naproxen medicines, the present invention therefore achieves more rapidly effective blood levels and thereby an accelerated onset of the analgesic effect. It thereby lessens the danger that the patient prematurely takes another tablet as a result of a too slow onset of the analgesic effect.

The elimination of lubricants and disintegrants and the reduction or elimination of further auxiliary agents in the tablet formulations according to the invention enables a significant decrease of the tablet weight and size. Since the quantity of sodium naproxen equivalent to 200 mg naproxen is only 220 mg, the weight difference to the insoluble naproxen is not too great, even if a sodium naproxen with about 12.5% by weight water (sodium dihydrate naproxen) is utilised in view of the better compressibility. In this case the quantity of sodium dihydrate naproxen equivalent to 200 mg naproxen is only 250 mg. Consequently, the tablets according to the invention are rapidly resorbed, as well as being comparatively small.

The expression "tablet core" indicates in the context of the present invention a tablet without sugar or film coat.

In the context of the present invention the stated water content of the sodium naproxen was determined in each case as loss on drying at 105°C. Thereby the water of crystallisation and further adsorbed water is completely lost.

The proportion of sodium naproxen in the tablet formulations according to the invention, is about 30 to 95% by weight, preferably about 60 to 95% by weight, and typically about 70 to 93% by weight, in particular about 70 to 85% by weight, based on the weight of the tablet core. Correspondingly the proportion of auxiliary agent in the tablet core is about 70 to 5% by weight, preferably about 40 to 5% by weight and typically about 30 to 7% by weight, in particular about 30 to 15% by weight.

According to one embodiment of the present invention, the tablet core can essentially consist of sodium naproxen and basic auxiliary agent. The proportion of basic auxiliary agent is at least about 5% by weight, based on the weight of the tablet core. Further the water content of the sodium naproxen in this embodiment should be preferably about 10 to 14% by weight, a water content of about 11 to 13% by weight, in particular about 11.5 to 12.5% by weight, being especially preferred. In addition the tablets should have a tablet hardness (measured by means of a Schleuniger Hardness tester) of preferably at least about 30 N, especially preferably at least about 40 N.

In general, it is however preferred to employ in the tablet core a low proportion of at least about 0.1% by weight of one or more further auxiliary agents in addition to one or more basic auxiliary agents, which is present in a total amount of at least about 5% by weight, based on the weight of the tablet core. The proportion of sodium naproxen is in this case preferably at most about 94.9% by weight, based on the weight of the tablet core.

In principle, the auxiliary agents that can be used in the tablet core can be water soluble or poorly water soluble or insoluble materials. For example, it can occasionally be desirable to use in the tablet mix an insoluble binding agent or an insoluble filler, native and microcrystalline celluloses, starches, modified starches, calcium phosphate and silicon oxide and/or a disintegrant, such as croscarmellose, crospovidone and crosslinked sodium carboxymethyl starch. In general, it is however preferred to use predominately or exclusively water soluble auxiliary agents in the tablet core. In the context of the present invention, "water soluble" describes those materials that are soluble in water at 25°C in a concentration of at least about 1% by weight. Especially preferred is the use of the water soluble auxiliary agent povidone as further auxiliary agent in addition to the basic auxiliary agent.

The total proportion of such auxiliary agents (which preferably can be water soluble), is, including the basic auxiliary agent, about 5 to 70% by weight, preferably about 20 to 40% by weight, and in particular about 25 to 35% by weight, based on the weight of the tablet core. The proportion of sodium naproxen in the tablet core can thereby preferably be about 30 to 93% by weight, especially preferably about 60 to 80% by weight and in particular about 65 to 75% by weight.

Preferably suitable as the auxiliary agent in the tablet core beside the basic auxiliary agents are fillers. Furthermore, if desired, the tablet core can contain one or more ionic or non-ionic tensides, for example sodium lauryl sulphate, sodium dodecyl sulphate, polysorbate or saccharose monopalmitate. The proportion of tensides, if present, can be for example about 0.1 to 10% by weight, based on the weight of the tablet core, however it preferably does not lie over about 5% by weight.

Preferably suitable basic auxiliary agents are such materials that give, in a concentration of 1% by weight in water at 25°C, an aqueous solution or suspension with a pH value of at least 7.5. Examples of suitable basic auxiliary agents are basic alkali metal salts, basic alkaline earth metal salts and basic ammonium salts, for example in the form of the carbonates, hydrogen carbonates, phosphates, hydrogen phosphates, oxides, hydroxides, citrates, tartrates, acetates or propionates, in particular basic sodium salts, basic potassium salts and basic ammonium salts, such as sodium hydrogen carbonate, potassium hydrogen carbonate, sodium carbonate, potassium carbonate, ammonium carbonate, trisodium citrate, disodium tartrate, dipotassium tartrate, magnesium oxide, calcium oxide, magnesium hydroxide, calcium hydroxide, magnesium carbonate, calcium carbonate, disodium hydrogen phosphate, dipotassium hydrogen phosphate, trisodium phosphate, tripotassium phosphate, tricalcium phosphate, sodium acetate, potassium acetate, sodium propionate etc., basic amino acids, such as lysine and arginine, and the like. In general, the water soluble, basic auxiliary agents such as sodium hydrogen carbonate, potassium hydrogen carbonate, sodium carbonate, potassium carbonate, trisodium citrate and trisodium phosphate are preferred. Especially preferably used are sodium hydrogen carbonate, potassium hydrogen carbonate or a mixture of both, in particular sodium hydrogen carbonate.

The basic auxiliary agents aid the formation of a basic micro milieu on the tablet surface and thereby presumably counteract a rapid precipitation of the acid naproxen in the acidic stomach. The proportion of the basic auxiliary agent in the tablet core can preferably be 5 to 70% by weight, in particular about 10 to 30% by weight, based on the weight of the tablet core. Typically, about 15 to 25% by weight of basic auxiliary agent is mostly used, for example about 18 to 22% by weight.

As filler in the tablet core generally auxiliary agents that improve the compressibility are suitable. However preferred are in general neutral to weakly acidic fillers that improve the compressibility, preferably those that do not have a buffering effect. In the context of the present invention the expression "neutral to weakly acidic filler" comprises in particular fillers that, at a concentration of 1% by weight in water at 25°C, result in an aqueous solution or suspension with a pH value between 4 and 7.5. Preferably water soluble fillers are used. Examples of preferably suitable fillers are sugars such as saccharose, glucose, fructose and lactose, hexoses such as mannitol, xylitol, maltitol, and sorbitol, hydrolysed or enzymatically split starch such as maltodextrin, cyclodextrins such as β- and γ-cyclodextrin, non-crosslinked (water soluble) polyvinylpyrrolidone, polyvinyl alcohols, polyethylene glycols, polypropylene glycols, alkali metal salts, alkaline earth metal salts and ammonium salts of organic or inorganic acids, in particular sodium, potassium, magnesium and calcium salts such as sodium chloride, potassium chloride, magnesium chloride, sodium sulphate, potassium sulphate, magnesium sulphate, trimagnesium dicitrate, tricalcium dicitrate, calcium lactate, calcium gluconate, calcium hydrogen phosphate and the like. Especially preferred fillers are hexoses such as sorbitol and mannitol, non-crosslinked polyvinylpyrrolidone, maltodextrin and sodium chloride, in particular water soluble, non-crosslinked polyvinylpyrrolidone, which is apparently also suitable to delay the precipitation of the naproxen in the stomach. Povidones K25-K90 (BASF, Germany) such as Povidone K25 and Povidones K29-32 are, for example, suitable as water soluble, non-crosslinked polyvinylpyrrolidones.

The proportion of filler in the tablet core can, if present, in general amount to about 1 to 50 by weight, based on the weight of the tablet core. Preferred are generally about 3 to 30% by weight, in particular about 10 to 25% by weight and typically about 15 to 20% by weight.

The tablet formulation according to the invention can contain fillers and basic auxiliary agents or only basic auxiliary agents. If the tablet core contains fillers as well as basic auxiliary agents, the optimal quantity can occasionally be a little lower than the aforementioned quantities. Furthermore the total quantity of filler and basic auxiliary agents expediently amounts to at the most about 70% by weight, preferably at most about 40% by weight and especially preferably at most about 30% by weight, based on the weight of the tablet core.

According to an especially preferred embodiment, the tablet formulation according to the invention contains as the basic component sodium hydrogen carbonate and/or potassium hydrogen carbonate and as water soluble filler non-crosslinked polyvinylpyrrolidone. Preferably the formulation can contain, based on the weight of the tablet core, about 5 to 20% by weight, in particular about 7 to 15% by weight, of non-crosslinked polyvinylpyrrolidone and about 5 to 20% by weight, in particular about 12 to 18% by weight of sodium hydrogen carbonate and/or potassium hydrogen carbonate. Preferably the tablet core can contain in addition saccharose palmitate for example in a quantity of about 2.5% by weight. Preferably the tablet core can consist of sodium naproxen, non-crosslinked polyvinylpyrrolidone, sodium hydrogen carbonate and/or potassium hydrogen carbonate and, if desired, saccharose palmitate.

With this preferred embodiment the disintegration of the tablet core in the acidic stomach is accelerated through the hydrogen carbonates, since the tablets react like a effervescent tablet. The polyvinylpyrrolidone as auxiliary agent that prevents crystallisation supports the formation of oversaturated solutions of naproxen and delays its crystallisation. The effervescence effect in the presence of tensides leads to foam with a large surface area. The formation from massive agglomerates of precipitated naproxen to larger, poorly soluble naproxen crystal agglomerates are thereby avoided and thus its re-dissolution is accelerated with the reaching of the duodenum.

If desired, the tablet mixture can also therefore contain a tenside such as sodium dodecylsulfate as auxiliary agent. However, the proportion of tenside, if present, is in general not over about 5% by weight and can for example be about 0.1% to 5% by weight, preferably about 0.1 to 3 % by weight, typically about 2% by weight, based on the weight of the tablet core. The addition of a tenside is however not mandatory, which is why the tablet core according to the invention may preferably also be free of tenside. If the water content of the sodium naproxen is in the range between 0.05 and 6% by weight, a comparatively high proportion of auxiliary agent is in general indicated, in order to counteract the reduction of the tableting properties of the sodium naproxen. Therefore in this case in general a proportion of auxiliary agent, in particular filler and basic auxiliary agent, of about 30 to 50% by weight, based on the weight of the tablet core, is preferred.

The tablets according to the invention can contain the active ingredient sodium naproxen in conventional dosages, high doses also being possible due to the low proportion of auxiliary agent. Therefore the tablets according to the invention can contain for example about 110 mg to 660 mg of sodium naproxen (based on the water free sodium naproxen; corresponding to 100 mg to 600 mg naproxen), in which dosages in the range of about 220 mg to 440 mg are preferred.

In one embodiment the present invention provides a tablet comprising sodium naproxen, sodium hydrogen carbonate, microcrystalline cellulose, croscarmellose, talcum, and magnesium stearate. In another embodiment the present invention provides a tablet comprising 50 to 60 % by weight of sodium naproxen, 15 to 25 % by weight of sodium hydrogen carbonate, 15 to 25 % by weight of microcrystalline cellulose, 2 to 6 % by weight of croscarmellose, 1 to 5 % by weight of talcum, and 0.5 to 2.2 % by weight of magnesium stearate. In still another embodiment the present invention provides a tablet comprising 55 to 65 % by weight of sodium naproxen, 10 to 25 % by weight of sodium hydrogen carbonate, 2 to 15 % by weight of microcrystalline cellulose, 2 to 6 % by weight of croscarmellose, 1 to 5 % by weight of talcum and 0.5 to 2.2 % by weight of magnesium stearate. In yet another embodiment the present invention provides a tablet comprising 55 to 65 % by weight of sodium naproxen, 10 to 25 % by weight of sodium hydrogen carbonate, 5 to 10 % by weight of hydroxy propyl cellulose, 2 to 15 % by weight of microcrystalline cellulose, 2 to 6 % by weight of croscarmellose, 1 to 5 % by weight of talcum, and 0.5 to 2.2 % by weight of magnesium stearate.

The tablet formulations according to the invention can preferably be coated with a sugar or film coating, in which all customary sugar and film coating materials are in principle suitable as coating materials. The thickness of the coat is not critical; however in general the proportion of the coat, based on the weight of the tablet core, is only about 1 to 10% by weight, preferably about 3 to 6% by weight.

The tablets according to the invention can be produced by compressing a mixture of the sodium naproxen and the auxiliary agent component into tablet cores and, if desired, coating the tablet cores with a sugar or film coating. The tabletization can be carried out in a manner known per se with customary tablet presses. Likewise, a sugar or film coat can be applied in a manner known per se by conventional methods. In general it is preferred that, prior to tabletization, sodium naproxen is granulated in dry form, optionally together with the auxiliary agent or a part of the auxiliary agent. Preferably for tabletization a granulate with a granular size of about 0.25 to 1.25 mm, in particular about 0.4 to 1.0 mm can be used, or the tablet core of the tablets according to the invention can consist of a granulate with this granular size. If the sodium naproxen has a bulk volume of at least 0.4 ml/g the granulation can be dispensed with, if desired. To determine the bulk volume, a 250 ml measuring cylinder is carefully and slowly filled up, without shaking, with an exactly weighed quantity of substance. Lastly, the poured in substance is levelled of f, if necessary by using a hairbrush to level off the surface of the substance in the cylinder, and the volume of the substance is read off. The bulk volume is the quotient of the read off volume and the mass of the introduced substance.

Basic auxiliary agents, and fillers which may optionally be used can be admixed either before the granulation, or just be admixed to the final mixture directly prior to tabletization, or a part of both components can be employed in the granulation and the rest added to the final mixture. However, if the tablet contains filler as well as basic auxiliary agent, in general it is preferred, to add the filler already in the granulation and the basic auxiliary agent only in the final mixture.

The invention also concerns a method to achieve an accelerated onset of analgesic effect, comprising the production of the tablets according to the invention and the administration thereof to a patient suffering from pain.

The invention is further illustrated by the following examples. In the Examples, Kollidon CL (Hoechst, Germany) denotes a water insoluble, crosslinked polyvinylpyrrolidone; Povidone K25-K90 (BASF, Germany) denotes water soluble, non-crosslinked polyvinylpyrrolidones; Hypromellose 2910, 6 and 15 mPas (Shin Etsu, Japan) is a water soluble hydroxypropylmethyl cellulose; Magrogol 4000 and Magrogol 6000 (Hoechst, Germany) is a highly polymerised, waxy and water soluble polyethylene glycol with an average molecular weight of 4000 to 6000 respectively; and titane dioxide (Schweizerhalle, Switzerland) is a water insoluble white pigment.

### Example 1

a) 231.0 kg sodium naproxen were mixed homogenously in a conventional mixer with 30.0 kg Povidone K25 and 3.0 kg sodium lauryl sulphate for 10 minutes. This mixture was compacted in a roller compactor, and the compacted material was broken over a sieve with the mesh width of 1.0 mm. Portions with a granular size under 0.4 mm were once more compacted and broken.
   50.0 kg sodium hydrogen carbonate and 2.0 kg magnesium stearate, sieved through a sieve with mesh width of 0.71 mm, were mixed in a conventional mixer with the compacted material for 10 minutes. The obtained final mixture was compressed on a rotary press with 16 presses at an average hourly output of 50 000 tablets. The obtained oval, biconvex tablets had a weight of 316 mg, a length of 11.5 mm, a width of 7.5 mm and a height of 4.5 mm.
   To determine the hardness of the tablets, the necessary force to crush the tablet between the motorised jaws of a Schleuniger Hardness Tester was measured. The average hardness (mean from 10 measurements) was 58 N.
   The disintegration time of the tablets was measured by means of the disintegration method described in the European Pharmacopoeia, 4th edition, Chapter 2.9.1, page 191, using water (pH about 7) as disintegration medium. The average disintegration time of the tablets (mean from 6 measurements) was 5.2 minutes.
b) 316 kg of the obtained tablets were loaded in a Glatt Coater and sprayed with a solution of 3.5 kg Hypromellose 2910, 0.75 kg lactose monohydrate and 0.75 kg Magrogol 6000 in 10 kg water and 40 kg ethanol (96%) at a product temperature of 35°C to 42°C, and isolated. Under the same conditions, the isolated film tablet cores were sprayed with a suspension of 2.8 kg Hypromellose 2910, 3.6 kg lactose monohydrate, 1.0 kg Magrogol 4000 and 2.6 kg titane dioxide in 56 kg water and 24 kg ethanol (96%). The dried film tablets were treated with a polishing solution of 2 kg Magrogol 6000 and 17 kg water. The final weight of the film tablets was 333 mg. The film tablets contained 220 mg water free sodium naproxen, corresponding to 200 mg naproxen.

In an analogous way, film coatings were successfully implemented, which contained as film forming agent carrageenan, polyvinyl alcohol and hydroxypropylmethyl cellulose as well as customary softeners such as polyethylene glycols, triethyl citrate and triacetin.

### Example 2

As described in Example 1, 316 kg of the final mixture for tabletization was produced. In an analogous manner to Example 1, this was compressed to form oblong, biconvex tablets with break score on one side, and the tablets obtained were processed to film tablets as described in Example 1. The tablet cores had a weight of 632 mg, a length of 17.0 mm, a width of 8.0 mm, a height of 5.0 mm and a content of water free sodium naproxen of 440 mg (corresponding to 400 mg naproxen); the average hardness was 78 N and the average disintegration time was 5.7 minutes. The final weight of the film tablets was 666 mg.

### Examples 3-28

### a) The tablet formulations listed in Table 1 were produced in an analogous manner to Example 1a.

To produce the granulate, the sodium naproxen (with diverse water contents) was mixed with the excipients used in dry granulation (auxiliary agents A), if any, in a conventional mixer for 10 minutes, the obtained mixture or, as the case may be, the sodium naproxen used without auxiliary agents was compacted on a roller compactor, the compacted material was broken over a sieve with the mesh width of 1.0 mm, and portions with a granular size under 0.4 mm were once more compacted and broken. In Example 25, a sodium naproxen with a mean particle size of 0.1 - 0.2 mm and a bulk volume of 0.4 g/ml was used and without prior compaction this was.directly used for tabletting. In the Examples 20 - 22, a granulate with a granular size of 0.4 - 1:25 mm (Example 20), 0 - 0.25 mm (Example 21) or 0 - 1.25 mm (Example 22) was produced and used in tabletization.

The obtained granulate (granular size in the range of 0.4 to 1.0 mm, if not otherwise indicated) was mixed in a conventional mixer with auxiliary agents (auxiliary agents B), if any, for 10 minutes. The obtained final mixture was compressed on a rotary press with 16 presses at an average hourly output of 40 000 - 60 000 tablets. The obtained oval, biconvex tablets had a weight of 285-345 mg, a length of 11.5 mm, a width of 7.5 mm and a height of about 4.0 -5.0 mm.

The water content of the sodium naproxen used, the proportion of the sodium naproxen in the tablet formulation, as well as the auxiliary agents A and B used and their proportions in the tablet formulation are compiled in Table 1. To determine the hardness (crushing strength) of the tablets, the necessary force to crush the tablets between the motorised jaws of a Schleuniger Hardness Tester was measured. The values reported in Table 1 are in each case the mean of 10 measurements.

**Table 1**

| Example | % by weight of water ^{a)} | % by weight of Na Naproxen ^{h)} | % by weight of auxiliary agent(s) A ^{b)} | % by weight of auxiliary agent(s) B ^{c)} | Tablet hardness [N] | Disintegration time [min] |
|---|---|---|---|---|---|---|
| 3 | 13.8% | 95.0% | - | 5.0% NaHCO₃ | 36 | 3.2 |
| 4 | 0.1% | 94.0% | - | 5.0% NaHCO₃, 1.0% Mg-Stearate | 31 | 3.8 |
| 5 | 8.3% | 94.5% | - | 5.0% NaHCO₃, 0.5% Mg-Stearate | 32 | 4.4 |
| 6 | 11.4% | 93.5% | - | 5.0% NaHCO₃, 1.5% Mg-Stearate | 27 | 7.4 |
| 7 | 0.3% | 95.0% | - | 5% NaHCO₃ | 22 | 4.2 |
| 8 | 3.3% | 85.6% | 8.9% Povidone K25 | 5.0% NaHCO₃, 0.5% Mg-Stearate | 48 | 5.8 |
| 9 | 1.8% | 83.5% | 7.5% Povidone K25 | 9.0% KHCO₃ | 44 | 3.6 |
| 10 | 1.8% | 77.4% | 7.5% Povidone K25 | 15.1% Na₃-Citrate | 49 | 5.6 |
| 11 | 1.8% | 77.4% | 17.5% Povidone K25 | 5.1% Na₃PO₄ | 73 | 7.1 |
| 12 | 1.8% | 77.4% | 7.5% Povidone K25 | 15.1% Na₂CO₃ | 54 | 6.9 |
| 13 | 1.8% | 77.4% | 7.5% Povidone K25 | 7.5% NaHCO₃, 7.6% KHCO₃ | 53 | 5.2 |
| 14 | 1.8% | 76.0% | 7.5% Povidone K25, 1.0% Na-dodecyl sulphate | 14.9% NaHCO₃, 0.6% Mg-Stearate | 66 | 5.0 |
| 15 | 12.7% | 67.3% | 7.6% Povidone K25 | 25.1% NaHCO₃ | 76 | 5.0 |
| 16 | 6.8% | 73.7% | 12.0% Maltodextrin | 4.3% PVP, 10.0% NaHCO₃ | 60 | 5.6 |
| 17 | 1.8% | 66.0% | 4.0% Povidone K25 | 10.5% microcryst. cellulose, 10.8% NaHCO₃, 3.7% Talc, 5.0% Crospovidone | 64 | 5.7 |
| 18 | 8.3% | 53.2% | 14.0% Povidone K25, 1.5% sodium lauryl sulphate, 3.5% Saccharose palmitate | 7% Maltodextrin 20.8% NaHCO₃ | 77 | 6.6 |
| 19 | 3.8% | 31.0% | 10.5% PVP K25, 37.5% NaHCO₃ | 1.0% Mg-Stearate, 20.0% NaHCO₃ | 59 | 4.8 |
| 20 | 1.8% | 64.9% | 8.0% Povidone K25 ^{d)}, 2.4% sodium lauryl sulphate | 9.7% NaHCO₃, 10% microcryst. cellulose, 4% Crospovidone, 1% Mg-Stearate | 73 | 5.4 |
| 21 | 1.8% | 64.9% | 8.0% Povidone K25 ^{e)}, 2.4% sodium lauryl sulphate | 9.7% NaHCO₃, 10% microcryst. cellulose, 4% Crospovidone, 1% Mg-Stearate | 35 | 8.1 |
| 22 | 1.8% | 64.9% | 8.0% Povidone K25 ^{f)}, 2.4% sodium lauryl sulphate | 9.7% NaHCO₃, 10% microcryst. cellulose, 4% Crospovidone, 1% Mg-Stearate | 58 | 6.3 |
| 23 | 2.7% | 67.3% | 18.0% Sorbitol | 9.7% NaHCO₃, 4% Maize starch, 1% Mg-Stearate | 71 | 6.0 |
| 24 | 10.8% | 75.1% | 7.3% Povidone K25 | 8.8% NaHCO₃, 8.8% NaCl | 62 | 5.9 |
| 25 | 2.7% | 43.0% ^{g)} | | 20.0% Povidone K25, 10.0% Mannitol, 22.0% NaHCO₃, 5.0% Talc | 87 | 6.2 |
| 26 | 0.1% | 55.0% | 12.5% NaHCO₃, 23.5% microcryst. cellulose, 4% Croscarmellose, 4.0% Talc 0.6% sodium lauryl sulphate | 1.4% Mg-Stearate | 85 | 7.2 |
| 27ⁱ⁾ | 9.5% | ca. 75.6% | ca. 19.0% microcryst. cellulose, ca. 2.4% Povidone K25 | ca. 1.0% Mg- Stearate ca. 2.0% Talc | 62 | 11.1 |
| 28 | 13.8% | 100.0% | - | - | 3.2 | 13.4 |

| | | | | | | |
|---|---|---|---|---|---|---|
| a) Water content of the sodium naproxen, measured as loss on drying at 105°C b) Auxiliary agent(s) in the sodium naproxen granulate c) Tabletization auxiliary agent(s) d) Granular size of the granulate in the range of 0.40 to 1.25 mm e) Granular size of the granulate in the range of 0 to 0.25 mm f) Granular size of the granulate in the range of 0 to 1.25 mm g) without compaction (sodium naproxen, bulk density 0.4 g/ml) h) % by weight of sodium naproxen refers to the active ingredient with the water content indicated in each case (Example: 70% by weight of sodium naproxen with 13.2% by weight water = 60.76% by weight sodium naproxen, water-free) i) sodium naproxen film tablet on the European market | | | | | | |

The disintegration time of the tablets was measured by means of the disintegration method described in the European Pharmacopoeia, 4th edition, Chapter 2.9.1, page 191, using water (pH about 7) as disintegration medium. The disintegration times listed in Table 1 are in each case the mean of 6 measurements.

The tablets according to the invention according to Example 3 can be produced in a slow running tablet press. The tablets only have a hardness of only 36 N and show sporadic tendency to capping. Interesting is their active ingredient release at pH 1.2 (Fig. 2) in comparison to the Examples 27 and 28. Examples 27 and 28 differentiate themselves from Example 3 through the fact that the tablets do not contain basic auxiliary agent. The superiority of the formulation according to the invention shows itself in the reduction of the disintegration time from 13.4 to 3.2 minutes and in particular with the dissolution test in artificial gastric juice (Fig. 2), where the oversaturation is favoured. An approximately 15 times higher concentration of dissolved naproxen (in oversaturated form, about 39%, instead of 2.5%) arises. This result is likewise confirmed by Example 27. Admittedly through the addition of microcrystalline cellulose and Povidone K25 a higher breaking strength and a marginally improved disintegration is achieved, however the oversaturation essential for the blood level increase is decisively reduced compared to the Example 3.

The tablets according to the Examples 3 -6 contain sodium naproxen with various drying losses. No addition of a lubricant is necessary with high drying losses. If too much magnesium stearate is added (Example 6), the tablet hardness falls under 30 N. Also the tablets according to Example 7 are no longer producible with sufficient hardness owing to the low water content of the sodium naproxen.

The tablets according to the Examples 8-16 result in all cases in sufficient tablet hardness and favourable disintegration times. The basic component can be compacted with the sodium naproxen, as well as also added to the final mixture. All basic auxiliary agents are shown to be suitable. Preferred are however basic auxiliary agents such as potassium hydrogen carbonate, sodium hydrogen carbonate, potassium carbonate and sodium carbonate. The positive influence of the Povidone K25 on sufficiently hard and abrasion resistant tablets is unmistakeable.

The tablets of the Examples 17, 20-23 and 26 contain basic auxiliary agent as well as insoluble fillers such as microcrystalline cellulose and disintegrants such as crospovidone, maize starch and croscarmellose. Beside Povidone K25, sorbitol was also found to be suitable, in order to be compacted together with sodium naproxen. The results with regard to hardness and disintegration times show that the microcrystalline cellulose only delivers an inconsiderable contribution for the achievement of harder tablets, and that its combination with typical disintegrants in comparison to expectations does not significantly foster the disintegration. This is associated apparently with the highly water soluble sodium naproxen which is present in excess.

Advantageous is the utilisation of tensides, such as sodium lauryl sulphate and saccharose monopalmitate or the combination of both tensides. In artificial gastric juice a very fine-pored foam arises through the interplay from escaping carbon dioxide and tenside, which prevents the agglomeration of the precipitated naproxen. Example 18 (Fig. 2) achieved correspondingly the highest oversaturation with almost 70% dissolved naproxen.

Example 19 illustrates a tablet with comparatively low active ingredient content of 31%. The polyvinylpyrrolidone and the active ingredient are preferably compacted in this case together with a fraction of the sodium hydrogen carbonate and the remainder of the basic component added to the final mixture.

In Example 25, mannitol and povidone aid the formation of a sodium naproxen mix, which is able to be tabletized well, which is further mixed with sodium hydrogen carbonate and talc and pressed to tablets. A pre-requisite for the direct pressing of the sodium naproxen to tablets is however its bulk density of at least 0.4 g/ml.

### Example 29 (Dissolution Test)

The active ingredient release from the tablets obtained in the preceding Examples was tested by means of the method described in the European Pharmacopoeia, 4th edition, Chapter 2.9.3, page 194, (Paddle Equipment) in 1000 ml of a 0.1 M hydrochloric acid (artificial gastric juice, pH 1.2).

The dissolution profiles of some formulations are graphically presented in Figure 2 for illustration. Figure 2 shows the dissolution profile, which was measured by the paddle method in 0.1 M hydrochloric acid at 50 rpm, of the non-coated tablets (tablet cores) according to Examples 1, 3, 18, 26, 27 and 28. Naproxen is an organic acid with a strong pH dependent solubility. In the pH range of 1-5 the solubility clearly lies under 0.1 g/l. Only above pH 6 does it greatly increase as a consequence of salt formation and it reaches a value of about 20 g/l at pH 7.4. If the in vitro release is measured at pH 7.4, it is not surprising that for all tablets, a rapid active ingredient release is observed. The in vitro test at pH 7.4 reveals however practically nothing about the behaviour in vivo, since the tablets firstly arrive in the acidic stomach and even in the upper small intestine pH values of 7.4 are unusually high. Therefore the release results at pH 7.4 corresponding Pharmacopoeia are not reported.

Surprisingly with the tablets according to the invention at pH 1.2 oversaturations with up to about 70% dissolved naproxen can be achieved, such as Examples 1, 18 and 26 illustrate. As well it should be expressly mentioned that the pH value of the dissolution medium in each case was practically not changed by the basic auxiliary agent and remained below pH 1.3.

The decline of the curves incipient after about 10-20 minutes is a consequence of the gradual crystallisation of the naproxen under in vitro conditions, whereby, the oversaturation is gradually broken down. It is certain that the oversaturation phenomenon plays an important role under in vivo resorption and that oversaturations are stabilised through the complex composition of the gastric and intestinal juices clearly better than under in vitro conditions.

In addition it must be taken into account that under in vivo conditions oversaturation phenomenon in the stomach are if anything strengthened through the use according to the invention of sodium naproxen with acid buffering basic auxiliary agents, as well as possible crystallisation delaying auxiliary agents, such as polyvinylpyrrolidone. In addition the resorption can be further accelerated through combination of CO₂-forming basic auxiliary agents with tenside, whereby a fine foam arises, which through its large surface very finely divides potentially precipitating, amorphous naproxen and rapidly goes into solution again with the higher pH values in the duodenum, whereby this is available for immediate resorption.

### Example 30

116.504 kg of sodium naproxen, 8.473 kg of crosscarmellose sodium, 2.118 kg of talc, 39.717 kg of sodium hydrogen carbonate and 37.811 kg of microcrystalline cellulose are weighed and filled through a vibration sieve of 1.5 mm mesh size into a 1200 L tank in this order. The ingredients are blended for 20 minutes with a speed of 6 rpm.

2.915 kg of magnesium stearate is weighed and sieved through a 1 mm sieve. It is added to the above described pre-blend. After the addition of magnesium stearate the pre-blend is blended for a further 5 minutes with the same blending speed.

After blending the pre-blend is compacted on an industrial scale roller compactor with a fixed gap-size. The compaction force is adjusted to 35-40 kN to achieve a suitable granule structure. The screen size of the granulator is a 1.0 mm sieve. Particles finer than 100 µm are separated by a vibration sieve and are directly recycled by a vacuum transport into the feeding hopper of the compactor and are compacted again.

After the compaction step the granules are blended for 5 minutes to make them homogeneous. After this, the remainder of talc (4.1 kg) and magnesium stearate (1.538 kg) are added to the granules and blended for additional 5 minutes.

The final blend is used for tabletting on a rotary tabletting machine. Tablets with a final weight of 400 mg and a hardness of 80-120 N are achieved.

After tabletting a coating solution is prepared by dispersing 6.144 kg of Opadry Blue HP into 24.576 kg of purified water: this solution is stirred for 2 hours before use. Into a coating pan, this solution is sprayed onto the cores until the tablet mass reaches 410 mg.

## Claims

1. Non-effervescent tablet for oral administration of sodium naproxen comprising a tablet core and, if desired, a sugar or film coat on the tablet core, wherein the tablet core consists of 30 to 95% by weight of sodium naproxen, based on the weight of the tablet core, and 70 to 5% by weight of auxiliary agent component, based on the weight of the tablet core, the auxiliary agent component comprising one or more basic auxiliary agents selected from basic alkali metal salts basic alkaline earth metal salts, basic ammonium salts and basic amino acids in a total quantity of at least 5%, based on the weight of the tablet core, wherein the sodium naproxen has a water content of 0.05 to 14% by weight, measured as loss on drying at 105°C.

2. Tablet as claimed in claim 1, wherein the tablet core consists of 60 to 95% by weight of sodium naproxen and 40 to 5% by weight of auxiliary agent component, based on the weight of the tablet core.

3. Tablet as claimed in any one of claims 1 to 2, wherein the tablet core consists of 70 to 93% by weight of sodium naproxen and 30 to 7% by weight of auxiliary agent component, based on the weight of the tablet core.

4. Tablet as claimed in any one of claims 1 to 3, wherein the sodium naproxen has a water content of 6 to 12.5% by weight.

5. Tablet as claimed in any one of claims 1 to 4, wherein the auxiliary agent component comprises one or more basic auxiliary agents in a total quantity of 10 to 30% by weight, based on the weight of the tablet core.

6. Tablet as claimed in any one of claims 1 to 5, wherein the auxiliary agent component comprises one or more basic auxiliary agents in a total quantity of 15 to 25°/O by weight, based on the weight of the tablet core.

7. Tablet as claimed in any one of the claims 1 to 6, wherein the basic auxiliary agent is water soluble.

8. Tablet as claimed in any one of claims 1 to 7, wherein the basic auxiliary agent is selected from sodium hydrogen carbonate, potassium hydrogen carbonate, sodium carbonate, potassium carbonate, trisodium citrate and trisodium phosphate.

9. Tablet as claimed in any one of claims 1 to 8, wherein the basic auxiliary agent is selected from sodium hydrogen carbonate and potassium hydrogen carbonate.

10. Tablet as claimed in any one of claims 1 to 9, wherein the auxiliary agent component comprises one or more neutral to weakly acidic fillers that improve the compressibility.

11. Tablet as claimed in any one of claims 1 to 10, wherein the auxiliary agent component comprises one or more water soluble, neutral to weakly acidic fillers that improve the compressibility.

12. Tablet as claimed in any one of claims 1 to 11, wherein the auxiliary agent component comprises one or more fillers, selected from sugars, hexoses, hydrolysed or enzymatically split starches, cyclodextrins, non-crosslinked polyvinylpyrrolidone, neutral to weakly acidic alkali metal salts, neutral to weakly acidic alkaline earth metal salts, and neutral to weakly acidic ammonium salts.

13. Tablet as claimed in any one of claims 1 to 12, wherein the auxiliary agent component comprises one or more fillers, selected from hexoses, non-crosslinked polyvinylpyrrolidone, maltodextrin and sodium chloride.

14. Tablet as claimed in any one of claims 1 to 13, wherein the auxiliary agent component comprises non-crosslinked polyvinyl pyrrolidone as filler.

15. Tablet as claimed in any one of claims 1 to 14, wherein the auxiliary agent component comprises one or more non-water soluble fillers that improve the compressibility and the tablet disintegration.

16. Tablet as claimed in any one of claims 1 to 15, wherein the auxiliary agent component comprises one or more fillers, selected from native and microcrystalline celluloses, starches, modified starches, calcium phosphates and silicon oxide.

17. Tablet as claimed in any one of claims 10 to 16, wherein the proportion of filler is 3 to 30% by weight, based on the weight of the tablet core.

18. Tablet as claimed in any one of claims 10 to 17, wherein the proportion of filler is 10 to 25% by weight, based on the weight of the tablet core.

19. Tablet as claimed in any one of claims 1 to 18, wherein the auxiliary agent component comprises at least one basic auxiliary agent, selected from sodium hydrogen carbonate and potassium hydrogen carbonate, and non-crosslinked polyvinylpyrrolidone as filler.

20. Tablet as claimed in any one of claims 1 to 19, wherein the auxiliary agent component comprises, based on the weight of the tablet core, 5 to 20% by weight of basic auxiliary agent, selected from sodium hydrogen carbonate and potassium hydrogen carbonate, and 5 to 20% by weight of non-crosslinked polyvinylpyrrolidone as filler.

21. Tablet as claimed in any one of claims 1 to 20, wherein the auxiliary agent component comprises a disintegrant.

22. Tablet as claimed in any one of claims 1 to 21, wherein the auxiliary agent component comprises a disintegrant selected from croscarmellose, crospovidone and crosslinked sodium carboxymethyl starch.

23. Tablet as claimed in any one of claims 1 to 22, wherein the auxiliary agent component comprises one or more lubricants and/or glidants.

24. Tablet as claimed in any one of claims 1 to 20, wherein the tablet core does not contain any lubricant and does not contain any glidant.

25. Tablet as claimed in any one of claims 1 to 24, wherein the auxiliary agent component contains one or more ionic or non-ionic tensides.

26. Tablet as claimed in any one of claims 1 to 25, wherein the auxiliary agent component contains one or more tensides, selected from sodium lauryl sulphate, sodium dodecyl sulphate, polysorbate and saccharose monopalmitate.

27. Tablet as claimed in claim 25 to 26, wherein the proportion of tenside is 0.1 to 5% by weight, based on the weight of the tablet core.

28. Tablet as claimed in any one of claims 1 to 27, wherein the tablet core consists of a granulate with a granular size distribution from 0.25 to 1.25 mm.

29. Tablet as claimed in any one of the claims 1 to 28, wherein the hardness of the tablet core is at least 30 N.

30. Tablet as claimed in any one of the claims 1 to 29 with a content of sodium naproxen of 110 to 660 mg, based on the water-free sodium naproxen.

31. Tablet as claimed in any one of the claims 1 to 9 and 28 to 30, wherein the tablet core consists of sodium naproxen and basic auxiliary agent.

32. Tablet as claimed in claim 1, comprising sodium naproxen, sodium hydrogen carbonate, microcrystalline cellulose, croscarmellose, talc, and magnesium stearate.

33. Tablet as claimed in claim 32, comprising 50 to 60% by weight of sodium naproxen, 15 to 25% by weight of sodium hydrogen carbonate, 15 to 25% by weight of microcrystalline cellulose, 2 to 6% by weight of croscarmellose, 1 to 5 % by weight of talc, and 0.5 to 2.2% by weight of magnesium stearate.

34. Tablet as claimed in claim 32, comprising 55 to 65% by weight of sodium naproxen, 10 to 25% by weight of sodium hydrogen carbonate, 2 to 15% by weight of microcrystalline cellulose, 2 to 6% by weight of croscarmellose, 1 to 5 % by weight of talc, and 0.5 to 2.2% by weight of magnesium stearate.

35. Tablet as claimed in claim 34, comprising 55 to 65 % by weight of sodium naproxen, 10 to 25% by weight of sodium hydrogen carbonate, 5 to 10% by weight of hydroxyl propyl cellulose, 2 to 15 % by weight of microcrystalline cellulose, 2 to 6% by weight of croscarmellose, 1 to 5 % by weight of tale, and 0.5 to 2.2 % by weight of magnesium stearate.

## Patentansprüche

1. Nicht-brausende Tablette zur oralen Verabreichung von Natrium-Naproxen enthaltend einen Tablettenkern und, wenn gewünscht, eine Zucker- oder Filmbeschichtung um den Tablettenkern, wobei der Tablettenkern aus 30 bis 95 Gewichtsprozent Natrium-Naproxen, bezogen auf das Gewicht des Tablettenkerns, und 70 bis 5 Gewichtsprozent Hilfsstoff-Komponente, bezogen auf das Gewicht des Tablettenkerns, besteht, wobei die Hilfsstoff-Komponente einen oder mehrere basische Hilfsstoffe umfasst, die ausgewählt sind aus basischen Alkalimetallsalzen, basischen Erdalkalimetallsalzen, basischen Ammoniumsalzen und basischen Aminosäuren, in einer Gesamtmenge von mindestens 5%, bezogen auf das Gewicht des Tablettenkerns, wobei das Natrium-Naproxen einen Wassergehalt von 0,05 bis 14 Gewichtsprozent hat, der als Gewichtsverlust bei Trocknung bei 105°C gemessen wird.

2. Tablette gemäss Anspruch 1, wobei der Tablettenkern aus 60 bis 95 Gewichtsprozent Natrium-Naproxen und 40 bis 5 Gewichtsprozent Hilfsstoff-Komponente, bezogen auf das Gewicht des Tablettenkerns, besteht.

3. Tablette gemäss einem der Ansprüche 1 bis 2, wobei der Tablettenkern aus 70 bis 93 Gewichtsprozent Natrium-Naproxen und 30 bis 7 Gewichtsprozent Hilfsstoff-Komponente, bezogen auf das Gewicht des Tablettenkerns, besteht.

4. Tablette gemäss einem der Ansprüche 1 bis 3, wobei das Natrium-Naproxen einen Wassergehalt von 6 bis 12,5 Gewichtsprozent hat.

5. Tablette gemäss einem der Ansprüche 1 bis 4, wobei die Hilfsstoff- Komponente einen oder mehrere basische Hilfsstoffe in einer Gesamtmenge von 10 bis 30 Gewichtsprozent, bezogen auf das Gewicht des Tablettenkerns, umfasst.

6. Tablette gemäss einem der Ansprüche 1 bis 5, wobei die Hilfsstoff- Komponente einen oder mehrere basische Hilfsstoffe in einer Gesamtmenge von 15 bis 25 Gewichtsprozent, bezogen auf das Gewicht des Tablettenkerns, umfasst.

7. Tablette gemäss einem der Ansprüche 1 bis 6, wobei der basische Hilfsstoff wasserlöslich ist.

8. Tablette gemäss einem der Ansprüche 1 bis 7, wobei der basische Hilfsstoff ausgewählt ist aus Natriumhydrogencarbonat, Kaliumhydrogencarbonat, Natriumcarbonat, Kaliumcarbonat, Trinatriumcitrat und Trinatriumphosphat.

9. Tablette gemäss einem der Ansprüche 1 bis 8, wobei der basische Hilfsstoff ausgewählt ist aus Natriumhydrogencarbonat und Kaliumhydrogencarbonat.

10. Tablette gemäss einem der Ansprüche 1 bis 9, wobei die Hilfsstoff-Komponente einen oder mehrere neutrale bis schwach saure Füllstoffe enthält, welche die Kompressibilität verbessern.

11. Tablette gemäss einem der Ansprüche 1 bis 10, wobei die Hilfsstoff-Komponente einen oder mehrere wasserlösliche, neutrale bis schwach saure Füllstoffe enthält, welche die Kompressibilität verbessern.

12. Tablette gemäss einem der Ansprüche 1 bis 11, wobei die Hilfsstoff-Komponente einen oder mehrere Füllstoffe enthält, die ausgewählt sind aus Zuckern, Hexosen, hydrolysierten oder enzymatisch gespaltenen Stärken, Cyclodextrinen, unvernetztem Polyvinylpyrrolidon, neutralen bis schwach sauren Alkalimetallsalzen, neutralen bis schwach sauren Erdalkalimetallsalzen, und neutralen bis schwach sauren Ammoniumsalzen.

13. Tablette gemäss einem der Ansprüche 1 bis 12, wobei die Hilfsstoff-Komponente einen oder mehrere Füllstoffe, ausgewählt aus Hexosen,unvernetztem Polyvinylpyrrolidon, Maltodextrin und Natriumchlorid, enthält.

14. Tablette gemäss einem der Ansprüche 1 bis 13, wobei die Hilfsstoff-Komponente unvernetztes Polyvinylpyrrolidon als Füllstoff enthält.

15. Tablette gemäss einem der Ansprüche 1 bis 14, wobei die Hilfsstoff-Komponente einen oder mehrere nicht-wasserlösliche Füllstoffe enthält, welche die Kompressibilität und den Tablettenzerfall verbessern.

16. Tablette gemäss einem der Ansprüche 1 bis 15, wobei die Hilfsstoff-Komponente einen oder mehrere Füllstoffe, ausgewählt aus nativen und mikrokristallinen Cellulosen, Stärken, modifizierten Stärken, Kalziumphosphaten und Siliziumoxid umfasst.

17. Tablette gemäss einem der Ansprüche 10 bis 16, wobei der Anteil des Füllstoffs 3 bis 30 Gewichtsprozent, bezogen auf das Gewicht des Tablettenkerns, ist.

18. Tablette gemäss einem der Ansprüche 10 bis 17, wobei der Anteil des Füllstoffs 10 bis 25 Gewichtsprozent, bezogen auf das Gewicht des Tablettenkerns, ist.

19. Tablette gemäss einem der Ansprüche 1 bis 18, wobei die Hilfsstoff-Komponente mindestens einen basischen Hilfsstoff, ausgewählt aus Natriumhydrogencarbonat und Kaliumhydrogencarbonat, und unvernetztes Polyvinylpyrrolidon als Füllstoff umfasst.

20. Tablette gemäss einem der Ansprüche 1 bis 19, wobei die Hilfsstoff-Komponente, bezogen auf das Gewicht des Tablettenkerns, 5 bis 20 Gewichtsprozent basischen Hilfsstoff, ausgewählt aus Natriumhydrogencarbonat und Kaliumhydrogencarbonat; und 5 bis 20 Gewichtsprozent unvernetztes Polyvinylpyrrolidon als Füllstoff umfasst.

21. Tablette gemäss einem der Ansprüche 1 bis 20, wobei die Hilfsstoff-Komponente ein Sprengmittel umfasst.

22. Tablette gemäss einem der Ansprüche 1 bis 21, wobei die Hilfsstoff-Komponente ein Sprengmittel ausgewählt aus Croscarmellose, Crospovidon und vernetzter Natriumcarboxymethyl-Stärke, umfasst.

23. Tablette gemäss einem der Ansprüche 1 bis 22, wobei die Hilfsstoff-Komponente ein oder mehrere Fliesshilfsmittel und/oder Gleitmittel umfasst.

24. Tablette gemäss einem der Ansprüche 1 bis 20, wobei der Tablettenkern keine Fliesshilfsmittel und keine Gleitmittel enthält.

25. Tablette gemäss einem der Ansprüche 1 bis 24, wobei die Hilfsstoff-Komponente eines oder mehrere ionische oder nicht-ionische Tenside enthält.

26. Tablette gemäss einem der Ansprüche 1 bis 25, wobei die Hilfsstoff-Komponente eines oder mehrere Tenside, ausgewählt aus Natriumlaurylsulfat, Natriumdodecylsulfat, Polysorbat und Sacharosemonopalmitat, enthält.

27. Tablette gemäss Anspruch 25 oder 26, wobei der Anteil Tenside 0,1 bis 5 Gewichtsprozent, bezogen auf das Gewicht des Tablettenkerns, ist.

28. Tablette gemäss einem der Ansprüche 1 bis 27, wobei der Tablettenkern aus einem Granulat mit einer Korngrössenverteilung von 0,25 bis 1,25 mm besteht.

29. Tablette gemäss einem der Ansprüche 1 bis 28, wobei die Härte des Tablettenkerns mindestens 30 N beträgt.

30. Tablette gemäss einem der Ansprüche 1 bis 29 mit einem Natrium-Naproxen Gehalt von 110 bis 660 mg, bezogen auf wasserfreies Natrium-Naproxen.

31. Tablette gemäss einem der Ansprüche 1 bis 9 und 28 bis 30, wobei der Tablettenkern aus Natrium-Naproxen und basischem Hilfsstoff besteht.

32. Tablette gemäss Anspruch 1, umfassend Natrium-Naproxen, Natriumhydrogencarbonat, mikrokristalline Cellulose, Croscarmellose, Talk und Magnesiumstearat.

33. Tablette gemäss Anspruch 32, enthaltend 50 bis 60 Gewichtsprozent Natrium-Naproxen, 15 bis 25 Gewichtsprozent Natriumhydrogencarbonat, 15 bis 25 Gewichtsprozent mikrokristalline Cellulose, 2 bis 6 Gewichtsprozent Croscarmellose, 1 bis 5 Gewichtsprozent Talk, und 0,5 bis 2,2 Gewichtsprozent Magnesiumstearat.

34. Tablette gemäss Anspruch 32, umfassend 55 bis 65 Gewichtsprozent Natrium-Naproxen, 10 bis 25 Gewichtsprozent Natriumhydrogencarbonat, 2 bis 15 Gewichtsprozent mikrokristalline Cellulose, 2 bis 6 Gewichtsprozent Croscarmellose, 1 bis 5 Gewichtsprozent Talk, und 0,5 bis 2,2 Gewichtsprozent Magnesiumstearat.

35. Tablette gemäss Anspruch 34, umfassend 55 bis 65 Gewichtsprozent Natrium-Naproxen, 10 bis 25 Gewichtsprozent Natriumhydrogencarbonat, 5 bis 10 Gewichtsprozent Hydroxypropyl-Cellulose, 2 bis 15 Gewichtsprozent mikrokristalline Cellulose, 2 bis 6 Gewichtsprozent Croscarmellose, 1 bis 5 Gewichtsprozent Talk, und 0,5 bis 2,2 Gewichtsprozent Magnesiumstearat.

## Revendications

1. Comprimé non effervescent pour administration orale de naproxène sodique comprenant un noyau de comprimé et, le cas échéant, une dragéification ou un pelliculage sur le noyau de comprimé, dans lequel le noyau de comprimé consiste de 30 à 95% en poids de naproxène sodique, sur la base du poids du noyau de comprimé, et 70 à 5% en poids d'adjuvant, sur la base du poids du noyau de comprimé, ledit adjuvant comprenant un ou plusieurs adjuvants basiques sélectionnés parmi les sels basiques de métaux alcalins, les sels basiques de métaux alcalino-terreux, les sels basiques d'ammonium et les acides aminés basiques, dans une quantité totale d'au moins 5%, sur la base du poids du noyau de comprimé, dans lequel le naproxène sodique présente une teneur en eau de 0,05 à 14% en poids, ladite teneur étant mesurée comme perte au séchage à 105°C.

2. Comprimé selon la revendication 1, dans lequel le noyau de comprimé consiste de 60 à 95% en poids de naproxène sodique et de 40 à 5% en poids d'adjuvant, sur la base du poids du noyau de comprimé.

3. Comprimé selon la revendication 1 ou 2, dans lequel le noyau de comprimé consiste de 70 à 93% en poids de naproxène sodique et de 30 à 7% en poids d'adjuvant, sur la base du poids du noyau de comprimé.

4. Comprimé selon l'une quelconque des revendications 1 à 3, dans lequel le naproxène sodique présente une teneur en eau de 6 à 12,5% en poids.

5. Comprimé selon l'une quelconque des revendications 1 à 4, dans lequel l'adjuvant comprend un ou plusieurs adjuvants basiques en une quantité totale de 10 à 30% en poids, sur la base du poids du noyau de comprimé.

6. Comprimé selon l'une quelconque des revendications 1 à 5, dans lequel l'adjuvant comprend un ou plusieurs adjuvants basiques en une quantité totale de 15 à 25% en poids, sur la base du poids du noyau de comprimé.

7. Comprimé selon l'une quelconque des revendications 1 à 6, dans lequel l'adjuvant basique est soluble dans l'eau.

8. Comprimé selon l'une quelconque des revendications 1 à 7, dans lequel l'adjuvant basique est sélectionné parmi l'hydrogénocarbonate de sodium, l'hydrogénocarbonate de potassium, le carbonate de sodium, le carbonate de potassium, le citrate de trisodium et le phosphate de trisodium.

9. Comprimé selon l'une quelconque des revendications 1 à 8, dans lequel l'adjuvant basique est sélectionné parmi l'hydrogénocarbonate de sodium et l'hydrogénocarbonate de potassium.

10. Comprimé selon l'une quelconque des revendications 1 à 9, dans lequel l'adjuvant comprend un ou plusieurs agents de remplissage neutres à faiblement acides qui améliorent la compressibilité.

11. Comprimé selon l'une quelconque des revendications 1 à 10, dans lequel l'adjuvant comprend un ou plusieurs agents de remplissage solubles dans l'eau, neutres à faiblement acides qui améliorent la compressibilité.

12. Comprimé selon l'une quelconque des revendications 1 à 11, dans lequel l'adjuvant comprend un ou plusieurs agents de remplissage, sélectionnés parmi les sucres, les hexoses, les amidons hydrolysés ou dégradés enzymatiquement, les cyclodextrines, la polyvinylpyrrolidone non réticulée, les sels de métaux alcalins neutres à faiblement acides, les sels de métaux alcalino-terreux neutres à faiblement acides, et les sels d'ammonium neutres à faiblement acides.

13. Comprimé selon l'une quelconque des revendications 1 à 12, dans lequel l'adjuvant comprend un ou plusieurs agents de remplissage, sélectionnés parmi les hexoses, la polyvinylpyrrolidone non réticulée, la maltodextrine, et le chlorure de sodium.

14. Comprimé selon l'une quelconque des revendications 1 à 13, dans lequel l'adjuvant comprend la polyvinylpyrrolidone non réticulée comme agent de remplissage.

15. Comprimé selon l'une quelconque des revendications 1 à 14, dans lequel l'adjuvant comprend un ou plusieurs agents de remplissage non solubles dans l'eau qui améliorent la compressibilité et la désintégration du comprimé.

16. Comprimé selon l'une quelconque des revendications 1 à 15, dans lequel l'adjuvant comprend un ou plusieurs agents de remplissage, sélectionnés parmi les celluloses naturelles et microcristallines, les amidons, les amidons modifiés, les phosphates de calcium et l'oxyde de silicium.

17. Comprimé selon l'une quelconque des revendications 10 à 16, dans lequel la proportion de l'agent de remplissage est de 3 à 30% en poids, sur la base du poids du noyau de comprimé.

18. Comprimé selon l'une quelconque des revendications 10 à 17, dans lequel la proportion de l'agent de remplissage est de 10 à 25% en poids, sur la base du poids du noyau de comprimé.

19. Comprimé selon l'une quelconque des revendications 1 à 18, dans lequel l'adjuvant comprend au moins un adjuvant basique, sélectionné parmi l'hydrogénocarbonate de sodium et l'hydrogénocarbonate de potassium, et de la polyvinylpyrrolidone non réticulée comme agent de remplissage.

20. Comprimé selon l'une quelconque des revendications 1 à 19, dans lequel l'ajduvant comprend, sur la base du poids du noyau de comprimé, 5 à 20% en poids d'adjuvant basique, sélectionné parmi l'hydrogénocarbonate de sodium et l'hydrogénocarbonate de potassium, et 5 à 20% en poids de polyvinylpyrrolidone non réticulée comme agent de remplissage.

21. Comprimé selon l'une quelconque des revendications 1 à 20, dans lequel l'adjuvant comprend un agent de délitement.

22. Comprimé selon l'une quelconque des revendications 1 à 21, dans lequel l'adjuvant comprend un agent de délitement sélectionné parmi la croscarmellose, la crospovidone et l'amidon carboxyméthylique de sodium réticulé.

23. Comprimé selon l'une quelconque des revendications 1 à 22, dans lequel l'adjuvant comprend un ou plusieurs lubrifiants et/ou agents de glissement.

24. Comprimé selon l'une quelconque des revendications 1 à 20, dans lequel le noyau de comprimé ne contient ni lubrifiant ni agent de glissement.

25. Comprimé selon l'une quelconque des revendications 1 à 24, dans lequel l'adjuvant contient un ou plusieurs tensioactifs ioniques ou non-ioniques.

26. Comprimé selon l'une quelconque des revendications 1 à 25, dans lequel l'adjuvant contient un ou plusieurs tensioactifs, sélectionnés parmi le laurylsulfate de sodium, le dodécylsulfate de sodium, le polysorbate et le monopalmitate de saccharose.

27. Comprimé selon la revendication 25 ou 26, dans lequel la proportion de tensioactif est de 0,1 à 5% en poids, sur la base du poids du noyau de comprimé.

28. Comprimé selon l'une quelconque des revendications 1 à 27, dans lequel le noyau de comprimé consiste d'un granulat ayant une distribution de la taille des granules entre 0,25 et 1,25 mm.

29. Comprimé selon l'une quelconque des revendications 1 à 28, dans lequel la dureté du noyau de comprimé est au moins de 30 N.

30. Comprimé selon l'une quelconque des revendications 1 à 29, ayant une teneur en naproxène sodique de 110 à 660 mg, sur la base du naproxène sodique déshydraté.

31. Comprimé selon l'une quelconque des revendications 1 à 9 et 28 à 30, dans lequel le noyau de comprimé consiste de naproxène sodique et d'un adjuvant basique.

32. Comprimé selon la revendication 1, comprenant du naproxène sodique, d'hydrogénocarbonate de sodium, de la cellulose microcristalline, de la croscarmellose, du talc, et du stéarate de magnésium.

33. Comprimé selon la revendication 32, comprenant 50 à 60% en poids de naproxène sodique, 15 à 25% en poids d'hydrogénocarbonate de sodium, 15 à 25% en poids de cellulose microcristalline, 2 à 6% en poids de croscarmellose, 1 à 5% en poids de talc, et 0,5 à 2,2% en poids de stéarate de magnésium.

34. Comprimé selon la revendication 32, comprenant 55 à 65% en poids de naproxène sodique, 10 à 25% en poids d'hydrogénocarbonate de sodium, 2 à 15% en poids de cellulose microcristalline, 2 à 6% en poids de croscarmellose, 1 à 5% en poids de talc, et 0,5 à 2,2% en poids de stéarate de magnésium.

35. Comprimé selon la revendication 34, comprenant 55 à 65% en poids de naproxène sodique, 10 à 25% en poids d'hydrogénocarbonate de sodium, 5 à 10% en poids de cellulose hydroxyle propyle, 2 à 15% en poids de cellulose microcristalline, 2 à 6% en poids de croscarmellose, 1 à 5% en poids de talc, et 0,5 à 2,2% en poids de stéarate de magnésium.
